# EUROPÄISCHE PATENTSCHRIFT

(11) **EP 0 489 327 B1**
(45) Veröffentlichungstag und Bekanntmachung des Hinweises auf die Patenterteilung: **27.05.1998**
(21) Anmeldenummer: 91120050.9
(22) Anmeldetag: 25.11.1991
(51) Int. Cl.: C07D 405/12, A61K 31/395

(54) **Chromanderivate**
Chroman derivatives
Dérivés de chromanne

(30) Priorität: 05.12.1990 DE 4038752
(43) Veröffentlichungstag der Anmeldung: 10.06.1992
(73) Patentinhaber: MERCK PATENT GmbH, D-64271 Darmstadt (DE)
(72) Erfinder: Gericke, Rolf, Dr., W-6104 Seeheim (DE); Baumgarth, Manfred, Dr., W-6100 Darmstadt (DE); Lues, Ingeborg, Dr., W-6100 Darmstadt (DE); Harting, Jürgen, Dr., W-6100 Darmstadt (DE); Bergmann, Rolf, Dr., W-6101 Reichelsheim (DE)

(56) Entgegenhaltungen:
- EP-A- 0 363 883
- GB-A- 2 204 868

## Beschreibung

Die Erfindung betrifft neue Chromanderivate der Formel I 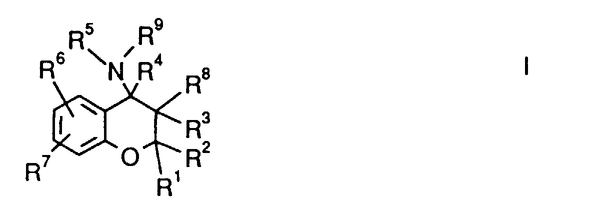 worin
- R¹ und R²: jeweils Methyl,
- R³: OH oder OAc,
- R⁴ und R⁷: H,
- R⁸ und R⁹: H oder Methyl,
- R⁵: unsubstituiertes oder einfach durch Methyl, Ethyl oder Isopropyl substituiertes Oxodihydropyridyl oder Oxodihydropyridazinyl,
- R⁶: CN,
- Ac: Acetyl
bedeuten,
sowie deren Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Ähnliche Verbindungen sind z.B. bekannt aus EP-A-363 883.

Es wurde gefunden, daß die Verbindungen der Formel I und ihre physiologisch unbedenklichen Salze bei guter Verträglichkeit wertvolle pharmakologische Eigenschaften besitzen. So zeigen sie Wirkungen auf das cardiovaskuläre System, wobei in der Regel bei niedrigeren Dosen ein selektiver Angriff am Coronarsystem, bei höheren ein blutdrucksenkender Effekt beobachtet werden kann. Am Coronoarsystem treten z.B. Widerstandsabnahme und Flußzunahme auf, wobei der Einfluß auf die Herzfrequenz gering bleibt. Weiterhin zeigen die Verbindungen eine relaxierende Wirkung auf verschiedene glattmuskuläre Organe (Gastrointestinaltrakt, Respirationssystem und Uterus). Die Wirkungen der Verbindungen können mit Hilfe an sich bekannter Methoden ermittelt werden, wie sie z.B. in EP-A-76075, EP-A-168619, EP-A-173848 oder AU-A-45547/85 (Derwent Farmdoc Nr. 86081769) sowie von K.S. Meesmann et al., Arzneimittelforschung 25 (11), 1975, 1770-1776, angegeben sind. Als Versuchstiere eignen sich z.B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

In den angegebenen Formeln bedeutet R¹ und R² vorzugsweise Methyl.

Falls R⁴ H bedeutet, ist R³ vorzugsweise OH, ferner bevorzugt O-CO-CH₃.

R⁵ ist bevorzugt unsubstituiertes 2-Oxo-1,2-dihydro-1- oder -3-pyridyl, 4-Oxo-1,4-dihydro-3-pyridyl oder aber substituiertes 1,2-Dihydro-2-oxo-4-pyridyl, besonders bevorzugt 1-Methyl-1,2-dihydro-2-oxo-4-pyridyl, ferner bevorzugt unsubstituiertes aber auch substituiertes 6-Oxo-1,6-dihydro-3-pyridazinyl, besonders bevorzugt durch Methyl-, Ethyl- oder Isopropyl in 1-Position substituiertes 6-Oxo-1,6-dihydro-3-pyridazinyl.

Diejenigen Reste R⁵, die eine zu einem Ring-N-Atom benachbarte Oxo-Gruppe enthalten, können auch in der tautomeren Lactamform vorliegen.
- R⁴ und R⁷: bedeuten vorzugsweise H.
- R⁶: ist bevorzugt CN.

Von den Resten R⁶ und R⁷ ist vorzugsweise der eine H, während der andere von H verschieden ist. Dieser andere Rest steht vorzugsweise in 6-Stellung, aber auch in 5-, 7- oder 8-Stellung, und ist vorzugsweise CN.

Der Rest R⁸ ist vorzugsweise H oder Methyl.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln Ia bis Ig ausgedrückt werden, die der Formel I entsprechen und worin die nicht näher bezeichneten Reste die bei der Formel I angegebene Bedeutung haben, worin jedoch
- in Ia: R¹ und R² jeweils CH₃ bedeuten;
- in Ib: R⁵ einen unsubstituierten oder einen durch A substituierten Oxo-dihydro-pyridyl- oder Oxo-dihydro-pyridazinylrest bedeutet;
- in Ic: R⁵ einen in 1-Position durch Methyl, Ethyl oder Isopropyl substituierten 6-Oxo-1,6-dihydro-3-pyridazinyl-rest bedeutet;
- in Ic: R⁵ einen 2-Oxo-1,2-dihydro-1-, -2-, -3- oder -4-pyridyl- oder einen 4-Oxo-1,4-dihydro-3-pyridyl-rest bedeutet;
- in Ie: R⁵ einen 1-Methyl-2-oxo-1,2-dihydro-4-pyridyl-rest bedeutet;
- in If: R¹ und R² jeweils CH₃,
R⁵ einen 1-Methyl-, 1-Ethyl- oder 1-Iso-propyl-6-oxo-1,6-dihydro-3-pyridazinyl-rest und
R⁹ H oder Methyl bedeuten.
- in Ig: R¹ und R² jeweils CH₃,
R⁵ 2-Oxo-1,2-dihydro-1-, -2-, -3- oder -4-pyridyl, 4-Oxo-1,4-dihydro-3-pyridyl oder 1-Methyl-2-oxo-1,2-dihydro-4-pyridyl und
R⁹ H oder Methyl bedeuten.

Weiterhin sind bevorzugt Verbindungen der Formeln I' sowie Ia' bis Ig', die den Formeln I sowie Ia bis Ig entsprechen, worin jedoch jeweils zusätzlich R³, OH oder OCOCH₃ und R⁴ H bedeuten, insbesondere solche Verbindungen der Formeln I' sowie Ia' bis Ig', worin jeweils zusätzlich R³ OH und R⁴ H bedeuten.

Ferner sind bevorzugt Verbindungen der Formeln I, I', Ia bis Ig sowie, Ia' bis Ig', worin jeweils zusätzlich
(a)
   - R⁶: CN bedeutet und in 6-Stellung steht und
   - R⁷: H bedeutet.

Insbesondere sind bevorzugt Verbindungen der Formeln I, I', Ia bis Ig, Ia' bis Ig', sowie die übrigen vorstehend als bevorzugt gekennzeichneten Gruppen von Verbindungen, worin zusätzlich R⁸ CH₃ bedeutet.

Im übrigen haben vor- und nachstehend die Reste R¹ bis R⁹, die bei Formel I angegebenen Bedeutungen, wenn nicht ausdrücklich etwas anderes angegeben ist.

Gegenstand der Erfindung ist ferner ein Verfahren zur Herstellung von Chromanderivaten der Formel I, dadurch gekennzeichnet, daß man ein Chroman der Formel II 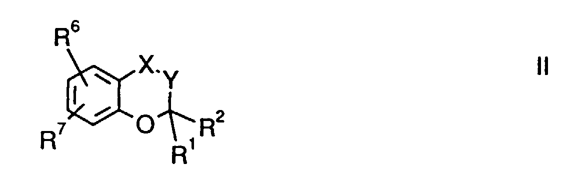 worin
- X-Y: 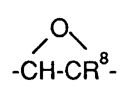 oder -CHE-CR³R⁸- und
- E: Cl, Br, I oder eine reaktionsfähig veresterte OH-Gruppe bedeuten und
R¹, R², R³, R⁶, R⁷ und R⁸ die bei Formel I angegebenen Bedeutungen haben,
mit einer Verbindung der Formel III

R⁵-NH-R⁹ III

worin
- R⁵ und R⁹: die bei Formel I angegebenen Bedeutungen haben,
oder mit einem ihrer reaktionsfähigen Derivate umsetzt,
und/oder daß man in einer Verbindung der Formel I einen Rest R3in einen anderen Rest R³ umwandelt,
und/oder daß man eine basische Verbindung der Formel I durch Behandeln mit einer Säure in eines ihrer Säureadditionssalze umwandelt.

Die Verbindungen der Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden die Verbindungen der Formel I durch Reaktion von Verbindungen der Formel II mit Verbindungen der Formel III hergestellt, zweckmäßig in Gegenwart eines inerten Lösungsmittels bei Temperaturen zwischen etwa 0 und 150°.

Ausgangsstoffe der Formel II mit X-Y = 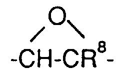 (3,4-Epoxychromane) sind bevorzugt.

Die Ausgangsstoffe II und III sind in der Regel bekannt (vgl. z.B. DE-OS 37 26 261). Sofern sie nicht bekannt sind, können sie nach an sich bekannten Methoden hergestellt werden. So sind die Ausgangsstoffe der Formel II 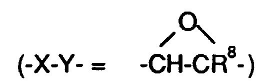 erhältlich durch Umsetzung von 2-Hydroxyacetophenonen der Formel 2-HO-R⁶R⁷C₆H₂-COCH₃ mit Ketonen der Formel R¹-CO-R² zu entsprechenden 4-Chromanonen der Formel IVa 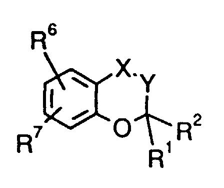
IVa -X-Y- = -CO-CH₂-
IVb -X-Y - = -CO-C(=CH-R⁹)-
IVc -X-Y- = -CHOH-CHR⁸-
IVd -X-Y- = -CH=CR⁸-
IVe-X-Y- = -CHBr-CR⁸OH-
gegebenenfalls Kondensation mit Aldehyden der Formel A-CHO (A = Alkyl mit 1-6 C-Atomen) zu 3-Alkyliden-4-chromanonen der Formel IVb, Reduktion z.B. mit NaBH₄ zu Chromanolen der Formel IVc, Dehydratisierung z.B. mit p-Toluolsulfonsäure, zu Chromenen der Formel IVd und Oxydation, z.B. mit 3-Chlorperbenzoesäure. Die letztgenannte Oxydation kann auch mehrstufig erfolgen. So kann man, z.B. mit N-Bromsuccinimid in wäßriger Lösung zunächst die Bromhydrine der Formel IVe herstellen und aus diesen anschließend mit einer Base, z.B. Natronlauge, HBr abspalten.

Man kann die Chromene der Formel IVd auch erhalten durch Kondensation von Salicylaldehyden der Formel 2-HO-R⁶R⁷C₆H₂-CHO mit Ketonen der Formel R¹-CO-CH₂-R⁸ zu Hydroxyketonen der Formel 2-HO-R⁶R⁷C₆H₂-CH=CR⁸-CO-R¹, Umsetzung mit Organo-Li-Verbindungen der Formel R²-Li, nachfolgende Hydrolyse zu Diolen der Formel 2-HO-R⁶R⁷C₆H₂-CH=CR⁸-CR¹R²-OH und Cyclisierung unter Wasserabspaltung.

In Verbindungen der Formel II (-X-Y- = -CHE-CR³R⁸-) kommen als "reaktionsfähig-veresterte-OH-Gruppen" insbesondere die Ester mit Alkylsulfonsäuren (worin die Alkylgruppe 1-6 C-Atome enthält) oder mit Arylsulfonsäuren (worin die Arylgruppe 6-10 C-Atome enthält) in Betracht. Diese Verbindungen sind erhältlich aus den 4-Chromanolen der Formel IVc durch Umsetzung mit einem anorganischen Säurehalogenid wie PCl₃, PBr₃, SOCl₂ oder SOBr₂ oder mit einem Sulfonsäurechlorid wie Methan- oder p-Toluolsulfonsäurechlorid.

Als reaktionsfähige Derivate von III eignen sich die entsprechenden Salze, z.B. die Na- oder K-Salze, die auch in situ entstehen können.

Es ist zweckmäßig, in Gegenwart einer Base zu arbeiten. Als Basen eignen sich z.B. Alkalimetall- oder Erdalkalimetallhydroxide, -carbonate,-alkoholate, -hydride oder auch -amide wie NaOH, KOH, Ca(OH)₂, Na₂CO₃, K₂CO₃, Na- oder K-methylat, -ethylat oder tert.-butylat, NaH, KH, CaH₂, NaNH2, KNH2, ferner organische Basen wie Triethylamin oder Pyridin, die auch im Überschuß angewendet werden können und dann gleichzeitig als Lösungsmittel dienen.

Als inerte Lösungsmittel eignen sich insbesondere Alkohole wie Methanol, Ethanol, Isopropanol, n-Butanol oder tert.-Butanol; Ether wie Diethylether, Diisopropylether, Tetrahydrofuran oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Dimethylformamid (DMF), Dimethylacetamid oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eianen sich Gemische dieser Lösunasmittel untereinander.

Das Epoxid II 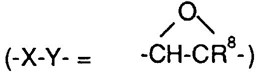 kann auch in situ hergestellt werden, z.B. durch Einwirkung einer Base auf das entsprechende Bromhydrin IVe.

Eine besonders bevorzugte Arbeitsweise besteht dann, daß man einen Alkohol (z.B. Ethanol) als Lösungsmittel verwendet und eine organische Base (z.B. Pyridin) hinzusetzt, wobei man zweckmäßig etwa 0,5 bis 20 Std. kocht.

Weiterhin kann man in einer Bindung der Formel I einen Rest R³ in einen anderen Rest R³ umwandeln.

Beispielsweise ist es möglich, daß man eine Hydroxygruppe acyliert.

Als acylierende Mittel zur Acylierung von Hydroxygruppen eignen sich zweckmäßig die Halogenide (z.B. Chloride oder Bromide) oder Anhydride von Carbonsäuren, z.B. Acetanhydrid, Propionylchlorid, Isobutyrylbromid, Ameisensäure/Essigsäureanhydrid, Benzoylchlorid. Der Zusatz einer Base wie Pyridin oder Triethylamin bei der Acylierung ist möglich. Man acyliert zweckmäßig in Gegenwart oder Abwesenheit eines inerten Lösungsmittels, z.B. eines Kohlenwasserstoffs wie Toluol, eines Nitril wie Acetonitril, eines Amids wie DMF oder eines Überschusses einer tertiären Base wie Pyridin oder Triethylamin bei Temperaturen zwischen etwa 0° und etwa 160°, vorzugsweise zwischen 20° und 120°. Eine Formylierung gelingt auch mit Ameisensäure in Gegenwart von Pyridin.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Aufreinigung der Verbindungen der Formel I verwendet werden.

Die Verbindungen der Formel I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Racemate, beispielsweise durch Umkristallisieren aus inerten Lösungsmitteln, in reiner Form isolieren kann. So haben z.B. Verbindungen der Formel I, worin R¹ = R², R³ = OH und R⁴ = H ist, zwei chirale Zentren; bei der Herstellung durch Reaktion von II mit III entsteht jedoch ganz überwiegend nur ein Racemat mit trans-Stellung der Substituenten R³ = OH und R⁵ R9N. Erhaltene Racemate können, falls erwünscht, nach an sich bekannten Methoden mechanisch, chemisch oder biochemisch in ihre Enantiomeren getrennt werden. So können aus dem Racemat durch Umsetzung mit einem optisch aktiven Trennmittel Diastereomere gebildet werden. Als Trennmittel für basische Verbindungen der Formel I eignen sich z.B. optisch aktive Säuren, wie die D- und L-Formen von Weinsäure, Dibenzoylweinsäure, Diacetylweinsäure, Camphansäure, Camphersulfonsäuren, Mandelsäure, Äpfelsäure oder Milchsäure, Carbinole (I, R³ = OH) können ferner mit Hilfe chiraler Acylierungsreagenzien, z.B. den angegebenen Säuren, insbesondere (+)- oder (-)-Camphansäure oder (+)- oder (-)-Campher-10-sulfonsäure, oder mit D- oder L-Methylbenzylisocyanat, verestert und dann getrennt werden (vgl. EP-A1-120428.). Die verschiedenen Formen der Diastereomeren können in an sich bekannter Weise, z.B. durch fraktionierte Kristallisation, getrennt, und die Enantiomeren der Formel I können in an sich bekannter Weise aus den Diastereomeren in Freiheit gesetzt werden. Enantiomerentrennungen gelingen ferner durch Chromatographie an optisch-aktiven Trägermaterialien.

Die Verbindungen der Formel I und ihre physiologisch unbedenklichen Salze können zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger- und Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z.B. zur Herstellung von Injektionspräparaten verwendet werden. Insbesondere für die topische Anwendung kommen auch liposomale Zubereitungen in Betracht. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der Therapie von Erkrankungen des menschlichen oder tierischen Körpers verwendet werden, insbesondere bei Therapie und/oder Prophylaxe von Störungen des cardiovasculären Systems, insbesondere dekompensierter Herzinsuffizienz, Angina pectoris, Arrhythmie, peripheren oder cerebralen Gefäßerkrankungen sowie Krankheitszuständen, die mit Bluthochdruck verbunden sind, ferner von Erkrankungen, die mit Veränderungen der nicht-vaskulären Muskulatur verbunden sind, z.B. Asthma, Inkontinenz der Harnblase.

Dabei werden die erfindungsgemäßen Substanzen in der Regel in Analogie zu bekannten Antianginosa bzw. Blutdrucksenkern, z.B. Nicorandil oder Cromakalim verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Die Verbindungen der Formel 1 und ihre Salze eignen sich, insbesondere bei topischer Anwendung, ferner zur Behandlung der Alopecia areata. Hierfür werden insbesondere pharmazeutische Zubereitungen verwendet, die zur topischen Behandlung der Kopfhaut geeignet und die oben genannt sind.

Sie enthalten etwa 0,005 bis 10, bevorzugt 0,5 bis 3 Gew.% mindestens einer Verbindung der Formel I und/oder mindestens eines ihrer Salze. Im übrigen können diese Verbindungen gegen Alopezie in Analogie zu den Angaben in der WO 88/00822 verwendet werden.

In den nachfolgenden Beispielen bedeutet "übliche Aufarbeitung", daß die Reaktionsmischung mit Wasser versetzt und mit organischen Lösungsmitteln wie z.B. Ethylacetat extrahiert wird und nach Abtrennung sowie Trocknung der organischen Phase über Natriumsulfat eine Reinigung durch Säulenchromatographie und/oder Kristallisation erfolgt:

Vor- und nachstehend sind sämtliche Temperaturen in °C angegeben.

### Beispiel 1

Zu einer Mischung von 1,2 g 3-Amino-1-methyl-1,6-dihydropyridazin-6-on und 0,3 g NaH (80%ig) in 50 ml Dimethylsulfoxid (DMSO) gibt man bei 25° 2,0 g 2,2-Dimethyl-3,4-epoxy-6-cyanchroman ("lla") und rührt 4 h. Übliche Aufarbeitung und Reinigung durch Säulenchromatographie (Ethylacetat/ Methanol) liefert 2,2-Dimethyl-4-(1-methyl-1,6-dihydro-6-oxo-3-pyridazinylamino)-6-cyan-3-chromanol, F. 117-119°.

Analog erhält man:
aus 3-N-Methylamino-1-methyl-1,6-dihydropyridazin-6-on und "IIa":
   2,2-Dimethyl-4-(1-methyl-1,6-dihydro-6-oxo-3-pyridazinyl-N-methylamino)-6-cyan-3-chromanol, F. 242-244°;
aus 3-Amino-1-isopropyl-1,6-dihydropyridazin-6-on und "IIa":
   2,2-Dimethyl-4-(1-isopropyl -1,6-dihydro-6-oxo-3-pyridazinyl-amino)-6-cyan-3-chromanol, F. 223-225°;
aus 3-Amino-1-ethyl-1,6-dihydropyridazin-6-on und "IIa":
   2,2-Dimethyl-4-(1-ethyl-1,6-dihydro-6-oxo-3-pyridazinyl-amino)-6-cyan-3-chromanol, F. 225-228°;
aus 3-Amino-1-methyl-1,6-dihydropyridazin-6-on und 2,2,3-Trimethyl-3,4-epoxy-6-cyan-chroman:
   2,2,3-Trimethyl-4-(1-methyl-1,6-dihydro-6-oxo-3-pyridazinyl-amino)-6-cyan-3-chromanol, F. 258-260°;
aus 3-Amino-1-methyl-1,6-dihydropyridazin-6-on und 2,2-Dimethyl-(3S,4S)-epoxy-6-cyan-chroman:
   2,2-Dimethyl-(4R)-(1-methyl-1,6-dihydro-6-oxo-3-pyridazinyl-amino)-6-cyan-(3S)-chromanol, F. 177-180°;
aus 4-Amino-1-methyl-1,2-dihydropyridin-2-on und 2,2-Dimethyl-(3S,4S)-epoxy-6-cyan-chroman:
   2,2-Dimethyl-(4R)-(1-methyl-1,2-dihydro-2-oxo-4-pyridazinyl-amino)-6-cyan-(3S)-chromanol, F. 283-285°.

### Beispiel 2

Eine Mischung von 1,1 g 1-Amino-1,2-dihydropyridin-2-on und 3,1 g 2,2,3-Trimethyl-3,4-epoxy-6-cyan-chroman wird auf Schmelztemperatur erhitzt und 8 h bei dieser Temperatur gerührt. Der Rückstand wird säulenchromatographisch gereinigt (Dichlormethan/Ethylacetat; Kieselgel). Man erhält 2,2,3-Trimethyl-4-(1,2-dihydro-2-oxo-1-pyridyl-amino)-6-cyan-3-chromanol, F. 184-187°.

Analog erhält man:
aus 1-Amino-1,2-dihydro-pyridin-2-on und "IIa":
   2,2-Dimethyl-4-(1,2-dihydro-2-oxo-1-pyridyl-amino)-6-cyan-3-chromanol, F. 202-204°.

### Beispiel 3

Zu einer Lösung von 1,1 g 3-Amino-1,6-dihydropyridin-6-on und 2,0 g "IIa" in 50 ml Ethanol gibt man 0,5 ml Triethylamin und kocht 2 h. Übliche Aufarbeitung liefert 2,2-Dimethyl-4-(1,6-dihydro-6-oxo-3-pyridyl-amino)-6-cyan-3-chromanol, F. 285-287°.

Analog erhält man aus 3-Amino-1,2-dihydropyridin-2-on und "IIa":
2,2-Dimethyl-4-(1,2-dihydro-2-oxo-3-pyridyl)-amino)-6-cyan-3-chromanol, F. 278-280°.

### Beispiel 4

Analog Beispiel 3 erhält man aus dem HCI-Salz des 3-Amino-1,4-dihydropyridin-4-ons und "lla" das 2,2-Dimethyl-4-(1,4-dihydro-4-oxo-3-pyridylamino)-6-cyan-3-chromanol; Hydrochlorid, F. 268-270°.

Die nachstehenden Beispiele betreffen pharmazeutische Zubereitungen, die Verbindungen der Formel I oder ihre physiologisch unbedenklichen Salze enthalten:

### Beispiel A Tabletten

Ein Gemisch von 1 g 2,2-Dimethyl-4-(1-methyl-1,6-dihydro-6-oxo-3-pyridazinyl-amino)-6-cyan-3-chromanol, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 0 ,1 mg Wirkstoff enthält.

### Beispiel B Dragees

Analog Beispiel A werden Tabletten gepreßt, die anschließend in üblicher Weise mit einem Überzug aus Saccharose, Kartofelstärke, Talk, Tragant und Farbstoff überzogen werden.

### Beispiel C Kapseln

Man füllt 1 kg 2,2,3-Trimethyl-4-(1-methyl-1,6-dihydro-6-oxo-3-pyridazinylamino)-6-cyan-3-chromanol in üblicher Weise in Hartgelatinekapseln, so daß jede Kapsel 0,5 mg Wirkstoff enthält.

### Beispiel D Ampullen

Eine Lösung von 10 g 2,2-Dimethyl-4-(1-methyl-1,6-dihydro-6-oxo-3-pyridazinyl-amino)-6-cyan-3-chromanol in 70 l 1,2-Propandiol wird mit zweifach destilliertem Wasser auf 100 1 aufgefüllt, steril filtriert, in Ampullen abgefüllt und steril verschlossen. Jede Ampulle enthält 0,1 mg Wirkstoff.

Analog sind Tabletten, Dragees, Kapseln oder Ampullen erhältlich, die einen oder mehrere der übrigen Wirkstoffe der Formel I und/oder ihrer physiologisch unbedenklichen Salze enthalten.

## Patentansprüche

1. Chromanderivate der Formel I 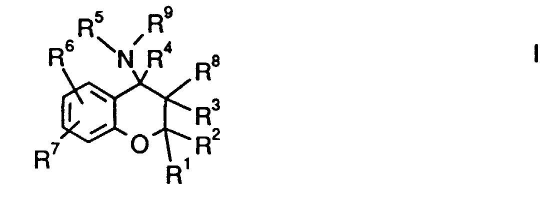 worin
R¹ und R² jeweils Methyl,
R³ OH oder OAc,
R⁴ und R⁷ H,
R⁸ und R⁹ H oder Methyl,
R⁵ unsubstituiertes oder einfach durch Methyl, Ethyl oder Isopropyl substituiertes Oxodihydropyridyl oder Oxodihydropyridazinyl,
R⁶ CN,
Ac Acetyl
bedeuten,
sowie deren Salze.

2. a) 2,2-Dimethyl-4-(1-methyl-1,6-dihydro-6-oxo-3-pyridazinylamino)-6-cyan-3-chromanol;
b) trans-3,4-Dihydro-4-(1,6-dihydro-1-methyl-6-oxo-3-pyridazinylamino)-2,2,3-trimethyl-6-cyan-3-chromanol;
c) (4R,3S)-2,2-Dimethyl-4-(1-methyl-1,6-dihydro-6-oxo-3-pyridazinyl-amino)-6-cyan-3-chromanol;
d) 2,2-Dimethyl-4-[N-(1-methyl-6-oxo-3-pyridazinyl)-N-methylamino]-6-cyan-3-chromanol.

3. Verfahren zur Herstellung von Chromanderivaten der Formel I gemäß Anspruch 1, dadurch gekennzeichnet, daß man ein Chroman der Formel II 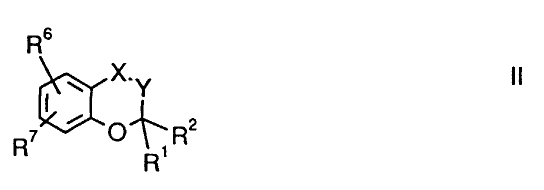 worin
X-Y 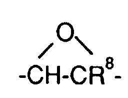 oder -CHE-CR³R⁸- und
E Cl, Br, I oder eine reaktionsfähig veresterte OH-Gruppe bedeuten und
R¹, R², R³, R⁶, R⁷ und R⁸ die bei Formel I angegebenen Bedeutungen haben, mit einer Verbindung der Formel III
R⁵-NH-R⁹ III
worin
R⁵ und R⁹ die bei Formel I angegebenen Bedeutungen haben,
oder mit einem ihrer reaktonsfähigen Derivate umsetzt,
und/oder daß man in einer Verbindung der Formel I einen Rest R³ in einen anderen Rest R³ umwandelt,
und/oder daß man eine basische Verbindung der Formel I durch Behandeln mit einer Säure in eines ihrer Säureadditionssalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I zur Bekämpfung von Krankheiten.

7. Verwendung von Verbindungen der Formel I zur Herstellung eines Arzneimittels.

## Claims

1. Chroman derivatives of the formula I 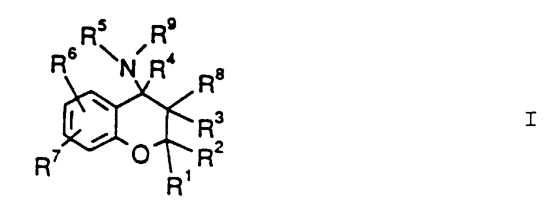 in which
R¹ and R² are each methyl,
R³ is OH or OAc,
R⁴ and R⁷ are H,
R⁸ and R⁹ are H or methyl,
R⁵ is oxodihydropyridyl or oxodihydropyridazinyl, which is unsubstituted or monosubstituted by methyl, ethyl or isopropyl,
R⁶ is CN,
Ac is acetyl,
and their salts.

2. a) 2,2-Dimethyl-4-(1-methyl-1,6-dihydro-6-oxo-3-pyridazinyl-amino)-6-cyano-3-chromanol;
b) trans-3,4-dihydro-4-(1,6-dihydro-1-methyl-6-oxo-3-pyridazinyl-amino)-2,2,3-trimethyl-6-cyano-3-chromanol;
c) (4R,3S)-2,2-dimethyl-4-(1-methyl-1,6-dihydro-6-oxo-3-pyridazinyl-amino)-6-cyano-3-chromanol;
d) 2,2-dimethyl-4-[N-(1-methyl-6-oxo-3-pyridazinyl)-N-methylamino]-6-cyano-3-chromanol.

3. Process for the preparation of chroman derivatives of the formula I according to Claim 1, characterised in that a chroman of the formula II 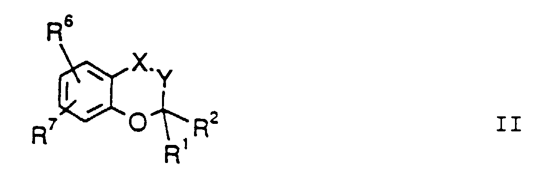 in which
X-Y is 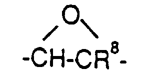 or -CHE-CR³R⁸- and
E is Cl, Br, I or a reactively esterified OH group and
R¹, R², R³, R⁶, R⁷ and R⁸ have the meanings given in formula I
is reacted with a compound of the formula III
R⁵-NH-R⁹ III
in which R⁵ and R⁹ have the meanings indicated in formula I,
or with one of its reactive derivatives,
and/or in that a radical R³ is converted into another radical R³ in a compound of the formula I,
and/or in that a basic compound of the formula I is converted into one of its acid addition salts by treating with an acid.

4. Process for the production of pharmaceutical preparations, characterised in that a compound of the formula I and/or one of its physiologically acceptable salts is brought into a suitable form for administration together with at least one solid, liquid or semi-liquid excipient or auxiliary.

5. Pharmaceutical preparation which contains at least one compound of the formula I and/or one of its physiologically acceptable salts.

6. Compounds of the formula I for the control of diseases.

7. Use of compounds of the formula I for the production of a medicament.

## Revendications

1. Dérivés de chromane de formule I 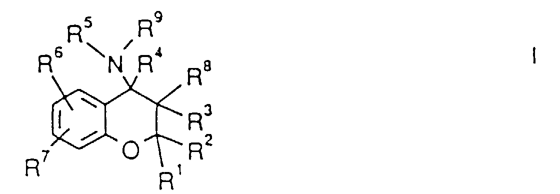 dans laquelle
R¹ et R² représentent chacun un méthyle,
R³ représente OH ou OAc
R⁴ et R⁷ représentent H,
R⁸ et R⁹ représentent H ou un méthyle,
R⁵ représente un oxodihydropyridyle ou un oxodihydropyridazinyle non substitué ou mono- substitué par un méthyle, éthyle ou isopropyle,
R⁶ représente CN,
Ac représente un acétyle,
ainsi que leurs sels.

2. a) 2,2-diméthyl-4-(1-méthyl-1,6-dihydro-6-oxo-3-pyridazinyl-amino)-6-cyano-3-chromanol;
b) trans-3,4-dihydro-4-(1,6-dihydro-1-méthyl-6-oxo-3-pyridazinyl-amino)-2,2,3-triméthyl-6-cyano-3-chromanol;
c) (4R,3S)-2,2-diméthyl-4-(1-méthyl-1,6-dihydro-6-oxo-3-pyridazinyl-amino)-6-cyano-3-chromanol;
d) 2,2-diméthyl-4-[N-(1-méthyl-6-oxo-3-pyridazinyl)-N-méthylamino]-6-cyano-3-chromanol.

3. Procédé de préparation de dérivés de chromane de formule I selon la revendication 1, caractérisé en ce qu'on fait réagir un chromane de formule II 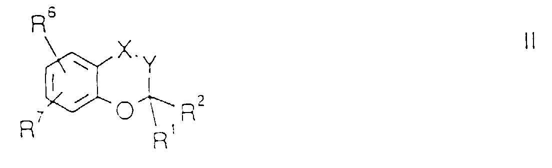 dans laquelle
X-Y représentent 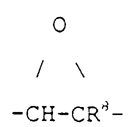 ou -CHE-CR³R⁸- et
E représente Cl, Br, I ou un groupe OH estérifié réactif et
R¹, R², R³, R⁶, R⁷ et R⁸ ont les significations indiquées pour la formule I,
avec un composé de formule III
R⁵-NH-R⁹ III
dans laquelle
R⁵ et R⁹ ont les significations indiquées dans la formule I,
ou avec un de ses dérivés réactifs,
et/ou en ce qu'on transforme dans un composé de formule I un radical R³ en un autre radical R³,
et/ou en ce qu'on transforme un composé basique de formule I en un de ses sels d'addition d'acides par traitement avec un acide.

4. Procédé de préparation de préparations pharmaceutiques, caractérisé en ce qu'on met sous une forme posologique appropriée un composé de formule I et/ou un de ses sels physiologiquement acceptables avec au moins un support ou additif solide, liquide ou semi-liquide.

5. Préparation pharmaceutique caractérisée en ce qu'elle contient au moins un composé de formule I et/ou un de ses sels physiologiquement acceptables.

6. Composés de formule I destinés à combattre les maladies.

7. Utilisation de composés de formule I pour la préparation d'un médicament.
